# EUROPEAN PATENT SPECIFICATION

(11) **EP 4 187 435 B1**
(45) Date of publication and mention of the grant of the patent: **11.12.2024**
(21) Application number: 20946488.2
(22) Date of filing: 14.08.2020
(51) Int. Cl.: G06F 18/214, G06F 18/2413, A61B 5/364, A61B 5/366, A61B 5/00, G16H 50/20, G16H 50/70

(54) **TRAINING METHOD AND MODEL FOR CONVOLUTIONAL NEURAL NETWORK BASED ON SAMPLE DISTRIBUTION IMPROVEMENT**
TRAININGSVERFAHREN UND -MODELL FÜR NEURONALES FALTUNGSNETZWERK AUF BASIS VON PROBENVERTEILUNGSVERBESSERUNG
PROCÉDÉ D'ENTRAÎNEMENT ET MODÈLE POUR RÉSEAU DE NEURONES CONVOLUTIF REPOSANT SUR UNE AMÉLIORATION DE LA DISTRIBUTION DES ÉCHANTILLONS

(30) Priority: 22.07.2020 CN 202010708023
(43) Date of publication of application: 31.05.2023
(73) Proprietor: Shanghai Sid Medical Co., Ltd, Shanghai 20000 (CN)
(72) Inventor: ZHU, Junjiang, Shanghai 20000 (CN); WANG, Yuxuan, Shanghai 20000 (CN); CHEN, Hongyan, Shanghai 20000 (CN); ZHU, Zhichao, Shanghai 20000 (CN); ZHANG, Shunyu, Shanghai 20000 (CN)
(74) Representative: Bayramoglu et al.
(86) International application number: PCT/CN2020/109294
(87) International publication number: WO 2022/016638

(56) References cited:
- WO-A1-2019/144311
- CN-A- 109 620 207
- CN-A- 109 620 207
- CN-A- 110 141 218
- US-A1- 2019 069 795
- FLORIAN LEHMANN ET AL: "Heartbeats in the Wild: A Field Study Exploring ECG Biometrics in Everyday Life", ARXIV.ORG, CORNELL UNIVERSITY LIBRARY, 201 OLIN LIBRARY CORNELL UNIVERSITY ITHACA, NY 14853, 6 March 2020 (2020-03-06), XP081615259, DOI: 10.1145/3313831.3376536
- ANTONIO H RIBEIRO ET AL: "Automatic diagnosis of the 12-lead ECG using a deep neural network", ARXIV.ORG, CORNELL UNIVERSITY LIBRARY, 201 OLIN LIBRARY CORNELL UNIVERSITY ITHACA, NY 14853, 2 April 2019 (2019-04-02), XP081642170, DOI: 10.1038/S41467-020-15432-4
- ZHOU FEI-YAN, JIN LIN-PENG, DONG JUN: "PVC Recognition Algorithm Based on Ensemble", ACTA ELECTRONICA SINICA, ZHONGGUO DIANZI XUEHUI, CN, vol. 45, no. 2, 25 February 2017 (2017-02-25), CN , pages 501 - 507, XP055891198, ISSN: 0372-2112, DOI: 10.3969/j.issn.0372-2112.2017.02.032

## Description

### TECHNICAL FIELD

The present disclosure relates to the technical field of heartbeat type screening in an artificial neural network, and in particular, to a method and model for training a convolutional neural network based on sample distribution improvement.

### BACKGROUND

An electrocardiogram (ECG) signal is a comprehensive reflection of electrical activities of numerous cardiac myocytes of a heart. Under a normal circumstance, cardiac myocytes of a sinoatrial node, an atrium, and a ventricle are depolarized in turn to form a heartbeat containing a signal segment of P, QRS, and T waves, and a plurality of heartbeats are combined to form an ECG signal segment. A premature ventricular beat is a heartbeat formed by depolarization of the ventricle due to an early electric impulse from an ectopic rhythm point of any part of the ventricle or a ventricular septum before an impulse of the sinoatrial node reaches the ventricle. It is very important to screen out all premature ventricular beats from a long-time ECG signal segment when determining a patient's condition, but this is time-consuming and labor-intensive. Therefore, it is of significance to screen out the premature ventricular beat from the ECG signal by using a computer. However, an electric shock can be generated from many positions in the heart, heartbeat shapes displayed in different electric shock positions are different, and there is a lot of interference. Therefore, during heartbeat classification, it is almost impossible to find all patterns and data of the premature ventricular beat and "other" types of heartbeats to train a deep learning model, which is easy to cause incorrect determination of a heartbeat type.

CN 109620207 A discloses method and apparatus for recognizing premature ventricular beat based on an improved convolutional neural network, which includes: pretreating an electrocardiosignal; truncating the electrocardiosignal as several heartbeat sequences, extracting RR spacing between each heartbeat and the prior heartbeat; calculating kurtosis value and deviation value of each heartbeat sequence; and recognizing heartbeat by inputting the heartbeat sequences, RR spacing, kurtosis value and deviation value into the improved convolutional neural network, to determine whether the heartbeat belongs to premature ventricular beat.

### SUMMARY

In order to resolve the shortcomings in the prior art, the present disclosure provides a method and model for training a convolutional neural network based on sample distribution improvement.

The present disclosure resolves the technical problems with following technical solutions:
A method for training a convolutional neural network based on sample distribution improvement includes following steps:
step S31: collecting enough lead-II heartbeat signals from a marked 12-lead ECG signal to form an initial dataset, where the lead-II heartbeat signals include a premature ventricular heartbeat signal and a non-premature ventricular heartbeat signal that each account for half of a total quantity of lead-II heartbeat signals; and setting a tag of the premature ventricular heartbeat signal to a, and a tag of the non-premature ventricular heartbeat signal to b;
step S32: taking statistics on all heartbeat data in the collected dataset to obtain an average value, a maximum value, a minimum value, a peak, and a kurtosis value of each piece of heartbeat data to form an average value array AVG={a1, a2, a3, ..., aN}, a maximum value array MAX={m1, m2, m3, ..., mN}, a minimum value array MIN={c1, c2, ..., cN}, a peak array F={f1, f2, ..., fN}, and a kurtosis value array Q={q1, q2, ..., qN}, and then calculating average values and standard deviations of the average value array, the maximum value array, the minimum value array, the peak array, and the kurtosis value array, which are denoted as *x̅_{avg}*, *σ_{avg}*, *x̅_{MAX}*, *σ_{MAX}*, *x̅_{MIN}*, *σ_{MIN}, x̅_{F}, σ_{F}, x̅_{Q},* and *σ_{Q}* respectively;
step S33: determining whether following conditions are satisfied for all heartbeat data in each piece of data: *x̅_{avg} -* 3*σ_{avg}* < *x_{avg}* < *x̅_{avg}* + 3*σ_{avg}*, *x̅*_{MAX} - 3*σ_{MAX}* < *x_{MAX}* < *x̅_{MAX}* + 3*σ_{MAX}*, *x̅_{MIN}* - 3*σ_{MIN}* < *x̅_{MIN}* < *x̅_{MIN}* + 3*σ_{MIN}*, *x̅_{F} -* 3*σ_{F}* < *x_{F}* < *x̅_{F}* + 3*σ_{F},* and *x̅_{Q}* -3*σ_{Q}* < *x_{Q} < x̅_{Q}* + 3*σ_{Q}*, obtaining a number of the conditions satisfied, and eliminating heartbeat data satisfying less than three of the above conditions in initial data to form a new heartbeat dataset;
step S34: repeating step S32 until a heartbeat dataset does not change, and using the heartbeat dataset as training data; and
step S35: inputting the training data and a corresponding tag to a convolutional neural network for training, to obtain a convolutional neural network model based on sample distribution improvement.

Preferably, according to the method for training a convolutional neural network based on sample distribution improvement, the lead-II heartbeat signal is sampled at a frequency of 500 Hz and filtered by a 0.5 Hz to 100 Hz Butterworth bandpass filter.

Preferably, according to the method for training a convolutional neural network based on sample distribution improvement, each heartbeat signal is an ECG signal from 0.38s before an R wave peak to 0.5s after the R wave peak.

Preferably, according to the method for training a convolutional neural network based on sample distribution improvement, the convolutional neural network model based on sample distribution improvement includes 10 network layers, namely, layers 1 to 9 and a classifier, where layers 1 to 5 each include a convolutional layer and a pooling layer, and use a LeakyReLU activation function; the convolutional layer in the layer 1 contains 32 kernels with a size of 7 each, and both a stride and a kernel size of the pooling layer in the layer 1 are 2; the convolutional layer in the layer 2 contains 32 kernels with a size of 5 each, and both a stride and a kernel size of the pooling layer in the layer 2 are 2; the convolutional layer in the layer 3 contains 32 kernels with a size of 3 each, and both a stride and a kernel size of the pooling layer in the layer 3 are 2; the convolutional layer in the layer 4 contains 32 kernels with a size of 5 each, and both a stride and a kernel size of the pooling layer in the layer 4 are 2; the convolutional layer in the layer 5 contains 32 kernels with a size of 5 each, and both a stride and a kernel size of the pooling layer in the layer 5 are 2; the convolutional layer in the layer 6 contains 32 kernels with a size of 5 each; the layer 7 is an input layer of a bidirectional long short term memory network layer, a quantity of neurons in the layer 7 is consistent with a quantity of output characteristics of the layer 6, and the bidirectional long short term memory network layer outputs 60 neurons in total, namely 30 neurons output forward and 30 neurons output backward; the layer 8 is an attention mechanism layer, which receives an output of the layer 7; and the layer 9 is a fully connected layer, which receives an output of the layer 8 and outputs 4 neurons.

Preferably, according to the method for training a convolutional neural network based on sample distribution improvement, a=1, b=0, and when an output value of the convolutional neural network model based on sample distribution improvement is greater than 0.5, a heartbeat of an unknown type is considered as a premature ventricular beat; or when an output value is less than or equal to 0.5, a heartbeat of an unknown type is considered as the non-premature ventricular heartbeat signal.

The present disclosure has following beneficial effects:
According to the method and model for training a convolutional neural network based on sample distribution improvement in the present disclosure, an average value *x_{avg}*, a maximum value *x_{MAX}*, a minimum value *x_{MIN}*, a peak *x_{F}*, and a kurtosis value *x_{Q}* of a heartbeat are calculated in advance, and whether the values satisfy conditions is determined to screen a sample. When heartbeat data satisfies less than three of *x̅_{avg}* - 3*σ_{avg}* < *x_{avg}* < *x̅_{avg}* + 3*σ_{avg}*, *x̅_{MAX}* - 3*σ_{MAX}* < *x_{MAX}* < *x̅_{MAX}* + 3*σ_{MAX}*, *x̅_{MIN}* - 3*σ_{MIN}* < *x_{MIN}* < *x̅_{MIN}* + 3*σ_{MIN}*, *x̅_{F}* - 3*σ_{F}*, < *x_{F}* < *x̅_{F}* + *3σ_{F}*, and *x̅_{Q}* - 3*σ_{Q}* < *x_{Q}* < *x̅_{Q}* + 3*σ_{Q}*, it is considered that training data used by the model does not conform to sample distribution, and a heartbeat signal failing to satisfy this requirement is eliminated, so as to reduce misjudgment of a heartbeat type.

### BRIEF DESCRIPTION OF THE DRAWINGS

The technical solutions of the present disclosure are described in further detail below with reference to the drawings and embodiments.
FIG. 1 is a flowchart of a method for identifying a heartbeat type of a convolutional neural network based on sample distribution improvement according to an embodiment of the present disclosure; and

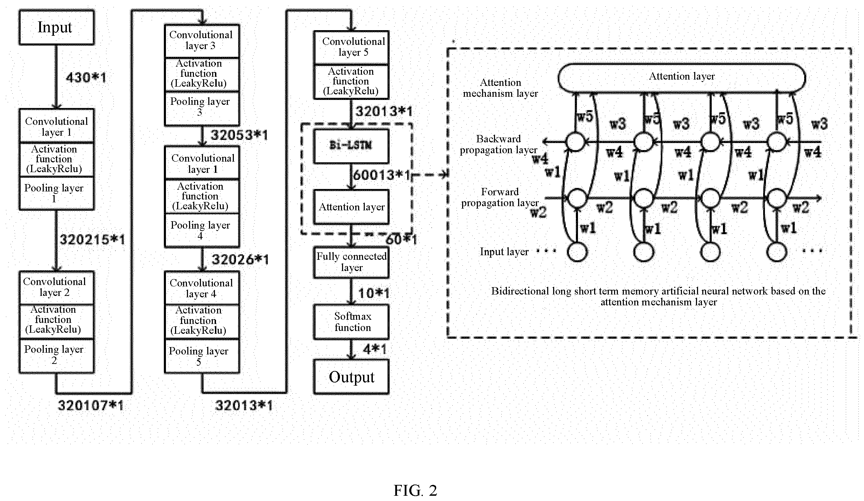

FIG. 2 is a schematic structural diagram of a convolutional neural network model based on sample distribution improvement according to an embodiment of the present disclosure.

### DETAILED DESCRIPTION OF THE EMBODIMENTS

It should be noted that the embodiments in the present disclosure and features in the embodiments may be combined with each other in a non-conflicting situation.

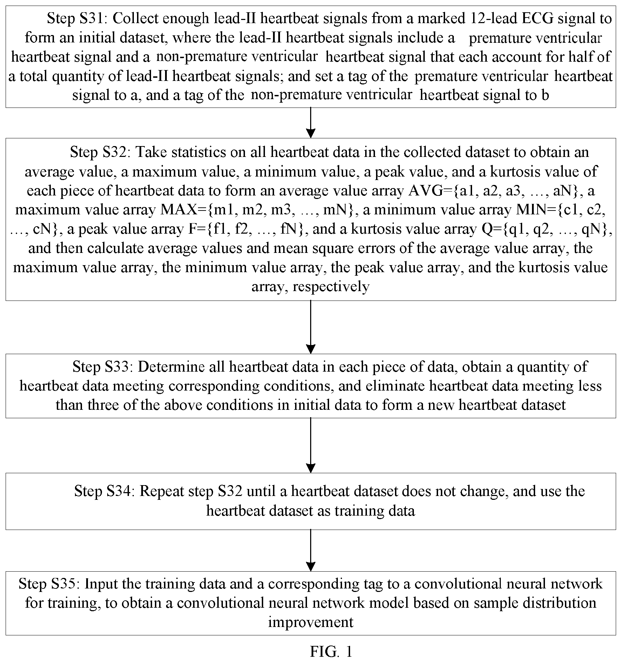

The technical solutions of the present disclosure will be described in detail below with reference to the drawings. As shown in FIG. 1, a method for training a convolutional neural network based on sample distribution improvement according to an embodiment includes following steps.

Step S31: Enough lead-II heartbeat signals are collected from a marked 12-lead ECG signal to form an initial dataset, where the lead-II heartbeat signals include a premature ventricular heartbeat signal and a non-premature ventricular heartbeat signal that each account for half of a total quantity of lead-II heartbeat signals; and a tag of the premature ventricular heartbeat signal is set to a, and a tag of the non-premature ventricular heartbeat signal is set to b.

Step S32: Statistics is taken on all heartbeat data in the collected dataset to obtain an average value, a maximum value, a minimum value, a peak, and a kurtosis value of each piece of heartbeat data to form an average value array A VG={a1, a2, a3, ..., aN}, a maximum value array MAX={m1, m2, m3, ..., mN}, a minimum value array MIN={c1, c2, ..., cN}, a peak array F={f1, f2, ..., fN}, and a kurtosis value array Q={q1, q2, ..., qN}, and then average values and standard deviations of the average value array, the maximum value array, the minimum value array, the peak array, and the kurtosis value array are calculated, which are denoted as *x̅_{avg}, σ_{avg}, x̅_{MAX}, σ_{MAX}, x̅_{MIN}, σ_{MIN}, x̅_{F}, σ_{F}, x̅_{Q},* and *σ_{Q}* respectively.

Step S33: Whether following conditions are satisfied is determined for all heartbeat data in each piece of data: *x̅_{avg}* - 3*σ_{avg}* < *x_{avg}* < *x̅_{avg}* + 3*σ_{avg}, x̅_{MAX}* - 3*σ_{MAX}* < *x_{MAX}* < *x̅_{MAX}* + 3*σ_{MAX}, x̅_{MIN}* - 3σ*_{MIN}* < *x_{MIN}* < *x̅_{MIN}* + 3*σ_{MIN}, x̅_{F} -* 3*σ_{F}* < *x_{F}* < *x̅_{F}* + 3*σ_{F}*, and *x̅_{Q}* - 3*σ_{Q}* < *x_{Q}* < *x̅_{Q}* + 3*σ_{Q}*, a number of the conditions satisfied is obtained, and heartbeat data satisfying less than three of the above conditions in initial data is eliminated to form a new heartbeat dataset.

Step S34: Step S32 is repeated until a heartbeat dataset does not change, and the heartbeat dataset is used as training data.

Step S35: The training data and a corresponding tag are input to a convolutional neural network for training, to obtain a convolutional neural network model based on sample distribution improvement.

The lead-II heartbeat signal in step S31 is sampled at a frequency of 500 Hz and filtered by a 0.5 Hz to 100 Hz Butterworth bandpass filter. The sampling frequency of the lead-II heartbeat signal needs to be adjusted to 500 Hz first if it is not 500 Hz. Each heartbeat signal is an ECG signal from 0.38s before an R wave peak to 0.5s after the R wave peak.

The convolutional neural network model based on sample distribution improvement includes 10 network layers, namely, layers 1 to 9 and a classifier. Layers 1 to 5 each include a convolutional layer and a pooling layer, and use a LeakyReLU activation function. The convolutional layer in the layer 1 contains 32 kernels with a size of 7 each, and both a stride and a kernel size of the pooling layer in the layer 1 are 2. The convolutional layer in the layer 2 contains 32 kernels with a size of 5 each, and both a stride and a kernel size of the pooling layer in the layer 2 are 2. The convolutional layer in the layer 3 contains 32 kernels with a size of 3 each, and both a stride and a kernel size of the pooling layer in the layer 3 are 2. The convolutional layer in the layer 4 contains 32 kernels with a size of 5 each, and both a stride and a kernel size of the pooling layer in the layer 4 are 2. The convolutional layer in the layer 5 contains 32 kernels with a size of 5 each, and both a stride and a kernel size of the pooling layer in the layer 5 are 2. The convolutional layer in the layer 6 contains 32 kernels with a size of 5 each. The layer 7 is an input layer of a bidirectional long short term memory network layer, a quantity of neurons in the layer 7 is consistent with a quantity of output characteristics of the layer 6, and the bidirectional long short term memory network layer outputs 60 neurons in total, namely 30 neurons output forward and 30 neurons output backward. The layer 8 is an attention mechanism layer, which receives an output of the layer 7. The layer 9 is a fully connected layer, which receives an output of the layer 8 and outputs 4 neurons.

Values of a and b may be set to 1 and 0 respectively. When an output value of the convolutional neural network model based on sample distribution improvement is greater than 0.5, a heartbeat of an unknown type is considered as a premature ventricular beat; or when an output value is less than or equal to 0.5, a heartbeat of an unknown type is considered as the non-premature ventricular heartbeat signal.

A convolutional neural network model is obtained through training by using the above-mentioned method for training a convolutional neural network. The convolutional neural network model can be used by performing the following steps:
Step S1: A heartbeat in a lead-II heartbeat signal of a 12-lead ECG signal of an unknown type is collected, and an average value *x_{avg}*, a maximum value *x_{MAX}*, a minimum value *x_{MIN}*, a peak *x_{F}*, and a kurtosis value *x_{Q}* of the heartbeat are calculated.
Step S2: A number of conditions satisfied is obtained, wherein the conditions are *x̅_{avg}* - 3*σ_{avg}* < *x_{avg}* < *x̅_{avg}* + 3*σ_{avg}*, *x̅_{MAX}* - 3*σ_{MAX}* < *x_{MAX}* < *x̅_{MAX}* + 3*σ_{MAX}*, *x̅_{MIN} -* 3*σ_{MIN}* < *x_{MIN}*, < *x̅_{MIN}*, + 3*σ_{MIN}*, *x̅_{F} -* 3*σ_{F}* < *x_{F}* < *x̅_{F}* + 3*σ_{F}*, and *x̅_{Q} -* 3*σ_{Q}* < *x_{Q}* < *x̅_{Q}* + 3*σ_{Q}.*
Step S3: If heartbeat data satisfies at least three of the above conditions in step S2, the heartbeat data is input to the convolutional neural network model based on sample distribution improvement in Embodiment 2 to determine a type of the heartbeat. Otherwise, it is considered that the heartbeat of the unknown type is a "type that cannot be determined".
Step S4: When an output value of the convolutional neural network model based on sample distribution improvement is approximate to a, the heartbeat of the unknown type is considered as a premature ventricular beat; or when an output value is approximate to b, the heartbeat of the unknown type is considered as a non-premature ventricular heartbeat signal.

Values of a and b may be set to 1 and 0 respectively. In step S4, when the output value of the convolutional neural network model based on sample distribution improvement is greater than 0.5, the heartbeat of the unknown type is considered as the premature ventricular beat; or when the output value is less than or equal to 0.5, the heartbeat of the unknown type is considered as the non-premature ventricular heartbeat signal. The technical scope of the present disclosure is subjected to the scope of the claims, and is not limited to the content of the specification.

Those skilled in the art should understand that the embodiments of the present disclosure may be provided as a method, a system, or a computer program product. Therefore, the present disclosure may use a form of hardware only embodiments, software only embodiments, or embodiments with a combination of software and hardware. Moreover, the present disclosure may be in a form of a computer program product that is implemented on one or more computer-usable storage media (including but not limited to a magnetic disk memory, a CD-ROM, an optical memory, and the like) that include computer-usable program code.

The present disclosure is described with reference to the flowcharts and/or block diagrams of the method, the device (system), and the computer program product according to the embodiments of the present disclosure. It should be understood that computer program instructions may be used to implement each process and/or each block in the flowcharts and/or the block diagrams and a combination of a process and/or a block in the flowcharts and/or the block diagrams. These computer program instructions may be provided for a general-purpose computer, a dedicated computer, an embedded processor, or a processor of another programmable data processing device to generate a machine, such that the instructions executed by a computer or a processor of another programmable data processing device generate an apparatus for implementing a specific function in one or more processes in the flowcharts and/or in one or more blocks in the block diagrams.

These computer program instructions may be stored in a computer-readable memory that can instruct a computer or another programmable data processing device to work in a specific manner, such that the instructions stored in the computer-readable memory generate an artifact that includes an instruction apparatus. The instruction apparatus implements a specific function in one or more processes in the flowcharts and/or in one or more blocks in the block diagrams.
These computer program instructions may alternatively be loaded onto a computer or another programmable data processing device, such that a series of operations and steps are performed on the computer or the another programmable device, thereby generating computer-implemented processing. Therefore, the instructions executed on the computer or the another programmable device provide steps for implementing a specific function in one or more processes in the flowcharts and/or in one or more blocks in the block diagrams.

## Claims

1. A method for training a convolutional neural network based on sample distribution improvement, comprising following steps:
a collection step (S31) of collecting enough lead-II heartbeat signals from a marked 12-lead electrocardiogram signal to form an initial dataset, wherein the lead-II heartbeat signals comprise a premature ventricular heartbeat signal and a non-premature ventricular heartbeat signal that each account for half of a total quantity of lead-II heartbeat signals; and setting a tag of the premature ventricular heartbeat signal to a, and a tag of the non-premature ventricular heartbeat signal to b;
a statistics step (S32) of taking statistics on all heartbeat data in the collected dataset to obtain an average value, a maximum value, a minimum value, a peak, and a kurtosis value of each piece of heartbeat data to form an average value array AVG={a1, a2, a3, ..., aN}, a maximum value array MAX={m1, m2, m3, ..., mN}, a minimum value array MIN={c1, c2, ..., cN}, a peak array F={f1, f2, ..., fN}, and a kurtosis value array Q={q1, q2, ..., qN}, and then calculating average values and standard deviations of the average value array, the maximum value array, the minimum value array, the peak array, and the kurtosis value array, which are denoted as *x̅_{avg}*, *σ_{avg}*, *x̅_{MAX}*, *σ_{MAX}*, *x̅_{MIN}*, *σ_{MIN}*, *x̅_{F}*, *σ_{F}*, *x̅_{Q}*, and *σ_{Q}* respectively;
an elimination step (S33) of determining whether following conditions are met for all heartbeat data in each piece of data: *x̅_{avg} -* 3*σ_{avg}* < *x_{avg}* < *x̅_{avg}* + 3*σ_{avg}, x̅_{MAX} -* 3*σ_{MAX}* < *x_{MAX}* < *x̅_{MAX}* + 3*σ_{MAX}*, *x̅_{MIN} -* 3*σ_{MIN}* < *x_{MIN}* < *x̅_{MIN}* + 3*σ_{MIN}, x̅_{F}* - 3*σ_{F}* < *x_{F}* < *x̅_{F}* + 3*σ_{F},* and *x̅_{Q} -* 3*σ_{Q}* < *x_{Q}* < *x̅_{Q}* + 3*σ_{Q}*, obtaining a number of the conditions satisfied, and eliminating heartbeat data satisfying less than three of the above conditions in initial data to form a new heartbeat dataset;
a repetition step (S34) of repeating the statistics step (S32) until a heartbeat dataset does not change, and using the heartbeat dataset as training data; and
an input step (S35) of inputting the training data and a corresponding tag to a convolutional neural network for training, to obtain a convolutional neural network model based on sample distribution improvement.

2. The method for training a convolutional neural network based on sample distribution improvement according to claim 1, wherein the lead-II heartbeat signal is sampled at a frequency of 500 Hz and filtered by a 0.5 Hz to 100 Hz Butterworth bandpass filter.

3. The method for training a convolutional neural network based on sample distribution improvement according to claim 1 or 2, wherein each heartbeat signal is an electrocardiogram signal from 0.38s before an R wave peak to 0.5s after the R wave peak.

4. The method for training a convolutional neural network based on sample distribution improvement according to claim 1 or 2, wherein the convolutional neural network model based on sample distribution improvement comprises 10 network layers, namely, layers 1 to 9 and a classifier, wherein layers 1 to 5 each comprise a convolutional layer and a pooling layer, and use a LeakyReLU activation function; the convolutional layer in the layer 1 contains 32 kernels with a size of 7 each, and both a stride and a kernel size of the pooling layer in the layer 1 are 2; the convolutional layer in the layer 2 contains 32 kernels with a size of 5 each, and both a stride and a kernel size of the pooling layer in the layer 2 are 2; the convolutional layer in the layer 3 contains 32 kernels with a size of 3 each, and both a stride and a kernel size of the pooling layer in the layer 3 are 2; the convolutional layer in the layer 4 contains 32 kernels with a size of 5 each, and both a stride and a kernel size of the pooling layer in the layer 4 are 2; the convolutional layer in the layer 5 contains 32 kernels with a size of 5 each, and both a stride and a kernel size of the pooling layer in the layer 5 are 2; the convolutional layer in the layer 6 contains 32 kernels with a size of 5 each; the layer 7 is an input layer of a bidirectional long short term memory network layer, a quantity of neurons in the layer 7 is consistent with a quantity of output characteristics of the layer 6, and the bidirectional long short term memory network layer outputs 60 neurons in total, namely 30 neurons output forward and 30 neurons output backward; the layer 8 is an attention mechanism layer, which receives an output of the layer 7; and the layer 9 is a fully connected layer, which receives an output of the layer 8 and outputs 4 neurons.

5. The method for training a convolutional neural network based on sample distribution improvement according to any one of claims 1 to 3, wherein a=1, b=0, and when an output value of the convolutional neural network model based on sample distribution improvement is greater than 0.5, a heartbeat of an unknown type is considered as a premature ventricular beat; or when an output value is less than or equal to 0.5, a heartbeat of an unknown type is considered as the non-premature ventricular heartbeat signal.

## Patentansprüche

1. Verfahren zum Trainieren eines Convolutional Neural Network basierend auf Probenverteilungsverbesserung, das die folgenden Schritte umfasst:
einen Sammelschritt (31) zum Sammeln von genügend Lead-II-Herzschlagsignalen von einem markierten 12-Lead-Elektrokardiogrammsignal, um einen ersten Datensatz zu bilden, wobei die Lead-II-Herzschlagsignale ein vorzeitiges ventrikuläres Herzschlagsignal und ein nicht vorzeitiges ventrikuläres Herzschlagsignal umfassen, wobei jedes die Hälfte der Gesamtmenge an Lead-II-Herzschlagsignalen ausmacht; und das Setzen eines Tags des vorzeitigen ventrikulären Herzschlagsignals auf a und eines Tags des nicht vorzeitigen ventrikulären Herzschlagsignals auf b;
einen Statistikschritt (S32) zum Erstellen von Statistiken über alle Herzschlagdaten in dem gesammelten Datensatz, um einen Mittelwert, einen Höchstwert, einen Mindestwert, einen Peak-Wert und einen Kurtosis-Wert jedes Teils von Herzschlagdaten zu erhalten, um ein Mittelwertfeld AVG={a1, a2, a3, ..., aN}, ein Höchstwertfeld MAX={m1, m2, m3, ..., mN}, ein Mindestwertfeld MIN={c1, c2, ..., cN}, ein Peak-Wertfeld F={f1, f2, ..., fN} und ein Kurtosis-Wertfeld Q={q₁, q2, ..., qN} zu bilden, und dann Mittelwerte und Standardabweichungen des Mittelwertfeldes, des Höchstwertfeldes, des Mindestwertfeldes, des Peak-Wertfeldes und des Kurtosis-Wertfeldes zu berechnen, die jeweils als *x̅_{avg}*, *σ_{avg}*, *x̅_{MAX}*, *σ_{MAX}, x̅_{MIN}*, *σ*_{*MIN*,} *x̅_{F}*, *σ_{F}*, *x̅_{Q}* und *σ_{Q}* bezeichnet werden;
einen Eliminationsschritt (S33) zum Bestimmen, ob folgende Bedingungen für alle Herzschlagdaten in jedem Teil der Daten erfüllt sind: *x̅_{avg} -* 3*σ_{avg}* < *x_{avg}* < *x̅_{avg}* + 3*σ_{avg} x̅_{MAX}* - 3*σ_{MAX}* < *x_{MAX}* < *x̅_{MAX}* + 3*σ_{MAX}*, *x̅_{MIN}* - 3*σ_{MIN}* < *x_{MIN}* < *x̅_{MIN}* + 3*σ_{MIN}*, *x̅_{F}* - *3σ_{F}* < *x_{F}* < *x̅_{F}* + *3σ_{F}* und *x̅_{Q} -* 3*σ_{Q}* < *x_{Q}* < *x̅_{Q}* + 3*σ_{Q}*, wobei eine Anzahl der erfüllten Bedingungen erhalten wird und Herzschlagdaten eliminiert werden, die weniger als drei der obigen Bedingungen in Anfangsdaten erfüllen, um einen neuen Herzschlagdatensatz zu bilden;
einen Wiederholungsschritt (S34) zum Wiederholen des Statistikschritts (S32), bis sich ein Herzschlagdatensatz nicht ändert, und Verwenden des Herzschlagdatensatzes als Trainingsdaten; und
einen Eingabeschritt (S35) zum Eingeben der Trainingsdaten und eines entsprechenden Tags in ein Convolutional Neural Network zum Trainieren, um ein Convolutional-Neural-Network-Modell basierend auf Probenverteilungsverbesserung zu erhalten.

2. Verfahren zum Trainieren eines Convolutional Neural Network basierend auf Probenverteilungsverbesserung nach Anspruch 1, wobei das Lead-II-Herzschlagsignal mit einer Frequenz von 500 Hz abgetastet und durch ein 0,5-Hz- bis 100-Hz-Butterworth-Bandpassfilter gefiltert wird.

3. Verfahren zum Trainieren eines Convolutional Neural Network basierend auf Probenverteilungsverbesserung nach Anspruch 1 oder 2, wobei jedes Herzschlagsignal ein Elektrokardiogrammsignal von 0,38 s vor einem R-Wellen-Peak bis 0,5 s nach dem R-Wellen-Peak ist.

4. Verfahren zum Trainieren eines Convolutional Neural Network basierend auf Probenverteilungsverbesserung nach Anspruch 1 oder 2, wobei das Convolutional-Neural-Network-Modells basierend auf Probenverteilungsverbesserung 10 Netzschichten umfasst, nämlich, Schichten 1 bis 9 und ein Klassifikator, wobei die Schichten 1 bis 5 jeweils eine Faltungsschicht und eine Poolingschicht umfassen, und Verwenden einer LeakyReLU-Aktivierungsfunktion; die Faltungsschicht in der Schicht 1 32 Kernel mit einer Größe von jeweils 7 enthält, und sowohl eine Schrittweite als auch eine Kernelgröße der Poolingschicht in Schicht 1 2 sind; die Faltungsschicht in Schicht 2 32 Kernel mit einer Größe von je 5 enthält, und sowohl eine Schrittweite als auch eine Kernelgröße der Poolingschicht in der Schicht 2 2 sind; die Faltungsschicht in der Schicht 3 32 Kernel mit einer Größe von je 3 enthält, und sowohl eine Schrittweite als auch eine Kernelgröße der Poolingschicht in Schicht 3 2 sind; die Faltungsschicht in Schicht 4 32 Kernel mit einer Größe von je 5 enthält, und sowohl eine Schrittweite als auch eine Kernelgröße der Poolingschicht in Schicht 4 2 sind; die Faltungsschicht in Schicht 5 32 Kernel mit einer Größe von je 5 enthält, und sowohl eine Schrittweite als auch eine Kernelgröße der Poolingschicht in Schicht 5 2 sind; die Faltungsschicht in Schicht 6 32 Kernel mit einer Größe von je 5 enthält; die Schicht 7 eine Eingangsschicht einer bidirektionalen Langzeit-Kurzzeitspeicher-Netzschicht ist, eine Menge an Neuronen in der Schicht 7 mit einer Menge an Ausgangseigenschaften der Schicht 6 übereinstimmt, und die bidirektionale Langzeit-Kurzzeitspeicher-Netzschicht insgesamt 60 Neuronen ausgibt, nämlich 30 Neuronen vorwärts ausgibt und 30 Neuronen rückwärts ausgibt; die Schicht 8 eine Aufmerksamkeitsmechanismusschicht ist, die eine Ausgabe der Schicht 7 empfängt; und die Schicht 9 eine vollständig verbundene Schicht ist, die eine Ausgabe der Schicht 8 empfängt und 4 Neuronen ausgibt.

5. Verfahren zum Trainieren eines Convolutional Neural Network basierend auf Probenverteilungsverbesserung nach einem der Ansprüche 1 bis 3, wobei a=1, b=0, und, wenn ein Ausgabewert des Convolutional-Neural-Network-Modells basierend auf Probenverteilungsverbesserung größer als 0,5 ist, ein Herzschlag eines unbekannten Typs als vorzeitiger ventrikulärer Herzschlag betrachtet wird; oder, wenn ein Ausgangswert kleiner oder gleich 0,5 ist, ein Herzschlag eines unbekannten Typs als nicht vorzeitiges ventrikuläres Herzschlagsignal betrachtet wird.

## Revendications

1. Procédé d'apprentissage d'un réseau neuronal convolutif sur la base d'une amélioration d'une distribution d'échantillons, comprenant les étapes suivantes :
une étape de collecte (31) consistant à collecter suffisamment de signaux de battement de coeur de la dérivation II à partir d'un signal d'électrocardiogramme à 12 dérivations marqué pour former un ensemble de données initial, dans lequel les signaux de battement de coeur de la dérivation II comprennent un signal de battement de coeur ventriculaire prématuré et un signal de battement de coeur non-ventriculaire prématuré qui représentent chacun la moitié d'une quantité totale de signaux de battement de coeur de dérivation II ; et à mettre une étiquette du signal de battement de coeur ventriculaire prématuré à a, et une étiquette du signal de battement de coeur ventriculaire non-prématuré à b ;
une étape de statistique (S32) consistant à faire des statistiques sur toutes les données de battement de coeur dans l'ensemble de données collectées pour obtenir une valeur moyenne, une valeur maximale, une valeur minimale, une valeur de crête et une valeur de kurtosis de chaque donnée de battement de coeur pour former une table de valeurs moyennes AVG={a1, a2, a3, ..., aN}, une table de valeurs maximales MAX={m1, m2, m3, ..., mN}, une table de valeurs minimales MIN={c1, c2, ..., cN}, une table de valeurs de crête F={f1, f2, ..., fN} et une table de valeurs de kurtosis Q={q1, q2, ..., qN}, puis calculer des valeurs moyennes et des écarts-types de la table de valeurs moyennes, de la table de valeurs maximales, de la table de valeurs minimales, de la table de valeurs de crête et de la table de valeurs de kurtosis, qui sont désignées par *x̅_{avg}*, *σ_{avg}*, *x̅_{MAX}*, *σ_{MAX}*, *x̅_{MIN}*, *σ_{MIN}*, *x̅_{F}*, *σ_{F}*, *x̅_{Q}* et *σ_{Q}* respectivement;
une étape d'élimination (S33) consistant à déterminer si les conditions suivantes sont satisfaites pour toutes les données de battement de coeur dans chaque donnée : *x̅_{avg} -* 3*σ_{avg}* < *x_{avg}* < *x̅_{avg}* + 3*σ_{avg}*, *x̅_{MAX}* - 3*σ_{MAX}* < *x_{MAX}* < *x̅_{MAX}* + 3*σ_{MAX}, x̅_{MIN}* - 3*σ_{MIN}* < *x_{MIN}* < *x̅_{MIN}* + 3*σ_{MIN}*, *x̅_{F}* - 3*σ_{F}* < *x_{F}* < *x̅_{F}* + 3*σ_{F}* et *x̅_{Q}* - 3*σ_{Q}* < *x_{Q}* < *x̅_{Q}* + 3*σ_{Q},* l'obtention d'un nombre de conditions satisfaites et l'élimination de données de battement de coeur satisfaisant à moins de trois des conditions ci-dessus dans les données initiales pour former un nouveau ensemble de données de battements de coeur ;
une étape de répétition (S34) consistant à répéter l'étape de statistique (S32) jusqu'à ce qu'un ensemble de données de battement de coeur ne change pas, et utiliser l'ensemble de données de battement de coeur comme données d'apprentissage ; et
une étape de saisie (S35) consistant à saisir les données d'apprentissage et une étiquette correspondante dans un réseau neuronal convolutif pour l'apprentissage, pour obtenir un modèle de réseau neuronal convolutif sur la base d'une amélioration de la distribution d'échantillon.

2. Procédé d'apprentissage d'un réseau neuronal convolutif sur la base d'une amélioration d'une distribution d'échantillons selon la revendication 1, dans lequel le signal de battement de coeur de la dérivation II est échantillonné à une fréquence de 500 Hz et filtré par un filtre passe-bande de Butterworth sur 0.5 Hz à 100 Hz.

3. Procédé d'apprentissage d'un réseau neuronal convolutif sur la base d'une amélioration d'une distribution d'échantillons selon la revendication 1 ou 2, dans lequel chaque signal de battement de coeur est un signal d'électrocardiogramme de 0,38 s avant un pic d'onde R à 0,5 s après le pic d'onde R.

4. Procédé d'apprentissage d'un réseau neuronal convolutif sur la base d'une amélioration d'une distribution d'échantillons selon la revendication 1 ou 2, dans lequel le modèle de réseau neuronal convolutif sur la base d'une amélioration d'une distribution d'échantillons comprend 10 couches de réseau, à savoir, les couches 1 à 9 et un classificateur, dans lequel les couches 1 à 5 comprennent chacune une couche de convolution et une couche de regroupement, et utilisent une fonction d'activation LeakyReLU ; la couche de convolution dans la couche 1 contient 32 noyaux avec une taille de 7 chacun, et à la fois un pas et une taille de noyau de la couche de regroupement dans la couche 1 sont 2 ; la couche de convolution dans la couche 2 contient 32 noyaux avec une taille de 5 chacun, et à la fois un pas et une taille de noyau de la couche de regroupement dans la couche 2 sont 2 ; la couche de convolution dans la couche 3 contient 32 noyaux avec une taille de 3 chacun, et à la fois un pas et une taille de noyau de la couche de regroupement dans la couche 3 sont 2 ; la couche de convolution dans la couche 4 contient 32 noyaux avec une taille de 5 chacun, et à la fois un pas et une taille de noyau de la couche de regroupement dans la couche 4 sont 2 ; la couche de convolution dans la couche 5 contient 32 noyaux avec une taille de 5 chacun, et à la fois un pas et une taille de noyau de la couche de regroupement dans la couche 5 sont 2 ; la couche de convolution dans la couche 6 contient 32 noyaux avec une taille de 5 chacun ; la couche 7 est une couche d'entrée d'une couche de réseau de mémoire à long-court terme bidirectionnelle, une quantité de neurones dans la couche 7 est cohérente avec une quantité de caractéristiques de sortie de la couche 6, et la couche de réseau de mémoire à long-court terme bidirectionnelle délivre en sortie 60 neurones au total, à savoir 30 neurones délivrés en sortie vers l'avant et 30 neurones délivrés en sortie vers l'arrière ; la couche 8 est une couche de mécanisme d'attention, qui reçoit une sortie de la couche 7 ; et la couche 9 est une couche entièrement connectée, qui reçoit une sortie de la couche 8 et délivre en sortie 4 neurones.

5. Procédé d'apprentissage d'un réseau neuronal convolutif sur la base d'une amélioration d'une distribution d'échantillons selon l'une quelconque des revendications 1 à 3, dans lequel a=1, b=0, et lorsqu'une valeur de sortie du modèle de réseau neuronal convolutif sur la base d'une amélioration d'une distribution d'échantillons est supérieure à 0,5, un battement de coeur d'un type inconnu est considéré comme un battement de coeur ventriculaire prématuré ; ou lorsqu'une valeur de sortie est inférieure ou égale à 0,5, un battement de coeur d'un type inconnu est considéré comme le signal de battement de coeur ventriculaire non-prématuré.
